# EUROPÄISCHE PATENTSCHRIFT

(11) **EP 0 971 620 B1**
(45) Veröffentlichungstag und Bekanntmachung des Hinweises auf die Patenterteilung: **31.07.2002**
(21) Anmeldenummer: 98909363.8
(22) Anmeldetag: 14.02.1998
(51) Int. Cl.: A61B 3/107, A61F 9/00

(54) **VERFAHREN ZUR BESTIMMUNG VON DATEN ZUR BEHANDLUNG DER HORNHAUT EINES AUGES**
METHOD FOR DETERMINING DATA FOR TREATING THE CORNEA
PROCEDE POUR DETERMINER DES DONNEES SERVANT AU TRAITEMENT DE LA CORNEE

(30) Priorität: 25.03.1997 DE 19712328; 02.04.1997 DE 19713623
(43) Veröffentlichungstag der Anmeldung: 19.01.2000
(73) Patentinhaber: TECHNOMED GESELLSCHAFT FÜR MED. UND MED.-TECHN.SYSTEME MBH, 52499 Baesweiler (DE)
(72) Erfinder: VON WALLFELD, Herbert, D-52428 Jülich (DE); NEUHANN, Thomas, D-80803 München (DE)
(74) Vertreter: Castell, Klaus, Dr.
(86) Internationale Anmeldenummer: DE9800426
(87) Internationale Veröffentlichungsnummer: WO9842291

(56) Entgegenhaltungen:
- WO-A-98/08048
- US-A- 4 669 466
- US-A- 5 384 608

## Beschreibung

Die Erfindung betrifft ein Verfahren zur Bestimmung von Daten zur Behandlung von Hornhaut eines Auges.

Bei der Behandlung der Hornhaut eines Auges wird üblicherweise das Brechungsverhalten der Hornhaut ermittelt, um anschließend in den meisten Fällen mit einem Laserverfahren durch Beschuss der Augenhornhaut die Krümmung und damit das Brechungsverhalten der Augenhornhaut zu verändern. Dabei ist es möglich, durch wenige gezielt gesetzte "Schüsse" eine Strukturveränderung der Hornhaut in bestimmten Bereichen zu erreichen oder Teile der Augenhornhaut abzutragen. Insbesondere durch radial unterschiedliche Intensität der Laserbehandlung wird somit das Brechungsverhalten der Hornhautoberfläche verändert.

Es hat sich jedoch herausgestellt, dass die Augenhornhaut über ihre gesamte Fläche meistens nicht ein einheitliches Brechungsverhalten hat und viele Verfahren nur bei einem über die Hornhaut verteilten, hinreichend gleichen Brechungsverhalten zu einem guten Ergebnis führen. Kleinere Partien der Augenhornhaut mit besonders hoher oder besonders geringer Krümmung werden daher nicht oder nur unzureichend korrigiert.

Aus der US 4,669,466 A und der US 5,384,608 A sind Verfahren bekannt, bei denen
- die Raumkoordinaten einer Vielzahl an Punkten der Oberfläche ermittelt werden,
- diese Raumkoordinaten als Datensatz in einen Rechner eingegeben werden,
- die Form und Lage einer Sollfläche in den Rechner eingegeben wird,
- für die Vielzahl an Punkten auf der Oberfläche der Abstand zur Sollfläche errechnet wird und
- die Raumkoordinaten und die Abstände der Vielzahl an Punkten als Datensatz zur Behandlung der Oberfläche ausgegeben werden.

Diese bekannten Verfahren legen eine Sollfläche fest und versuchen die Oberfläche so zu behandeln, dass die Unterschiede zur Sollfläche eliminiert werden. Problematisch ist jedoch, dass für eine beliebig geformte Oberfläche nicht eindeutig eine Sollfläche festgelegt werden kann. Eine optimale Sollfläche kann erst dann festgelegt werden, wenn die Abstände zur Oberfläche bekannt sind.

Der Erfindung liegt daher die Aufgabe zugrunde, ein gattungsgemäßes Verfahren weiterzubilden, um Orte unterschiedlichster Krümmung auf einer Oberfläche mit minimalen Eingriffen zu reduzieren, um eine für den individuellen Fall geeignete Oberflächenkrümmung zu erzielen.

Diese Aufgabe wird durch das in Anspruch 1 definierte Verfahren gelöst, wobei in weiteren Verfahrensschritten der berechnete Datensatz durch Rechnersimulation der eigentlichen Oberflächenbehandlung überprüft wird und bei zu großer Abweichung des Simulationsergebnisses von der angestrebten Oberflächengestalt solange die Form und/oder Lage der Sollfläche variiert wird und anhand dieser neuen Sollfläche eine erneute Abstandsberechnung erfolgt, bis ein optimales Ergebnis erreicht ist.

Der Erfindung liegt die Erkenntnis zugrunde, dass für eine beliebig geformte Oberfläche nicht eindeutig eine Sollfläche festgelegt werden kann. Erst durch mehrmaliges Ändern der Sollfläche und jeweilige Ermittlung der notwendigen Schnitte kann der Arzt ein Sollfläche festlegen, die als Grundlage für die weitere Behandlung dient.

Dieses erfindungsgemäße Verfahren erlaubt es, die Oberfläche nicht nur pauschal zu behandeln, sondern je nach Auflösung jeden einzelnen Punkt der Augenhornhaut mit seiner Position zu bestimmen, um anschließend durch Vergleich mit einer vorgegebenen Sollfläche den positiven oder negativen Abstand zur Sollfläche zu berechnen und für die eigentliche Behandlung der Oberfläche bereitzustellen. Dadurch können lokale regionale Überhöhungen intensiver behandelt werden, während von Natur aus vorhandene Senken einer geringeren oder gar keiner Behandlung zugeführt werden. Der durch das Verfahren zur Verfügung gestellte Datensatz ermöglicht somit eine gezielte Behandlung auch einer relativ unebenen Augenhornhaut, die durch das anschließende Behandlungsverfahren eine über die gesamte Fläche regelmäßige Krümmung erhalten soll.

Da die Form und/oder Lage der Sollfläche variierbar ist, ist es möglich, die Sollfläche am Computer im Vergleich mit der Oberfläche der Augenhornhaut auf der Grundlage klinischer Erfahrung festzulegen. Die vom Computer sofort errechneten Abweichungen zwischen Sollfläche und Augenhornhaut helfen dem Arzt die Sollfläche richtig zu bestimmen.

Das Verfahren eignet sich vor allem für spiegelnde Oberflächen, da diese mittels der Topometrie leicht vermessen werden können.

Eine einfache Methode zur Bestimmung der Orte der Vielzahl an Punkten der Oberfläche liegt darin, die Orte der Oberfläche der Vielzahl an Punkten topometrisch iterativ als Koordinaten zu berechnen. Eine interative Berechnungsmethode zur Berechnung der Koordinaten einzelner Orte einer spiegelnden Oberfläche ist in der PCT/DE95/01579 beschrieben, auf die vollinhaltlich Bezug genommen wird.

Um die Arbeit dem behandelnden Arzt zu erleichtern, ist es von Vorteil, wenn die Orte der Vielzahl an Punkten graphisch dargestellt werden. Dazu eignet sich beispielsweise eine Netzdarstellung, die deutlich zeigt, wie die Form der Augenhornhaut verläuft oder wie die Form von einer Kugelfläche abweicht.

Zum besseren Vergleich ist es vorteilhaft, wenn auch die Form und/oder Lage der Sollfläche graphisch dargestellt wird. Hierzu können die gleichen Verfahren verwendet werden, wobei es sich anbietet, die Oberflächen entweder nebeneinander oder in ihrer relativen Anordnung zueinander darzustellen.

Eine weitere Ausgestaltung der Erfindung sieht vor, daß auch die Abstände der Vielzahl an Punkten zur Sollfläche graphisch dargestellt werden. Auch dies kann in dreidimensionalen Darstellungen, Projektions- oder Schnittdarstellungen dem behandelnden Arzt als Graphik zur Verfügung gestellt werden.

Eine besonders einfache Verständlichkeit der Darstellung wird dadurch erreicht, dass unterschiedliche Abstände durch unterschiedliche Farben dargestellt werden. Dies ermöglicht es dem behandelnden Arzt, ohne einen aufwendigen Zahlenvergleich die Bereiche zu erkennen, die besonders weit von der Sollkurve abweichen.

Alternativ oder unterstützend zur Bestimmung der Sollfläche nach klinischer Erfahrung kann die Sollfläche auch durch Raytracing bestimmt werden. Dabei wird durch bestimmte Anforderungen an den Strahlenverlauf und insbesondere deren Schnittpunkt mit der Retina eine Sollkurve für die Augenhornhaut berechnet.

Eine Weiterentwicklung der Erfindung sieht vor, dass mit dem Datensatz zur Behandlung der Oberfläche ein Oberflächenbehandlungsverfahren simuliert wird und das Ergebnis angezeigt wird. Dazu werden in den Computer zusätzlich die Daten zu einem speziellen Oberflächenbehandlungsverfahren eingegeben, wonach der Computer das mit diesem Oberflächenbehandlungsverfahren erzielbare Ergebnis dem behandelnden Arzt anzeigt.

Wenn das Oberflächenbehandlungsverfahren bspw. Ein Laserverfahren ist, können die Daten der durch einen einzigen Schuss bewirkten Änderung der Gestalt der Hornhaut in den Computer eingegeben werden. Der Computer berechnet dann

Ort und Anzahl der notwendigen Schüsse zur Annäherung der Hornhautoberfläche an die Sollfläche. Sollte das Ergebnis nicht zufriedenstellend sein, kann entweder das Oberflächenbehandlungsverfahren geändert werden oder die Sollfläche wird in Ort oder Verlauf variiert. Der Computer erlaubt es, in kürzester Zeit verschiedene Behandlungsverfahren und Sollflächenvorgaben bis zur Anzeige des erzielbaren Ergebnisses zu simulieren, um anschließend am Patienten das optimale Behandlungsverfahren durchzuführen.

Ein Ausführungsbeispiel der Erfindung wird im folgenden anhand der Zeichnung näher erläutert.

Es zeigt,
- Figur 1: einen Schnitt durch eine Augenhornhaut mit überzeichneter Oberflächendeformation,
- Figur 2: eine Draufsicht auf eine Hornhaut mit ihren unterschiedlichen Krümmungsbereichen und
- Figur 3: einen Schnitt durch die Augenhornhaut gemäß Figur 1 nach einer Laserbehandlung.

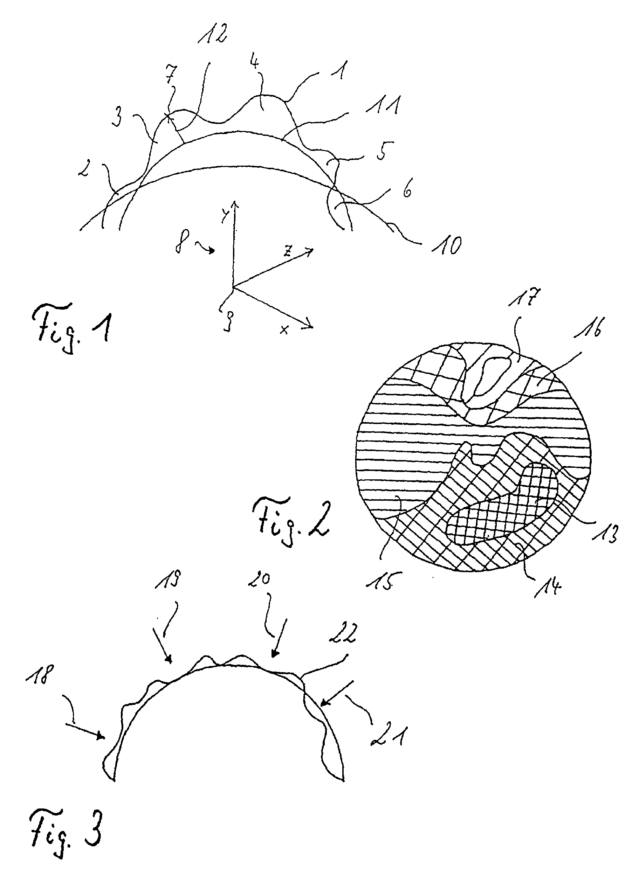

Die in Figur 1 gezeigte Hornhautoberfläche 1 ist mit stark überzeichneten Unebenheiten 2, 3, 4, 5 und 6 dargestellt. Als Beispiel für die Vielzahl an Punkten ist der Punkt 7 dargestellt, dessen Ortmittels der Koordinaten eines Koordinatensystems 8 mit dem beliebig festgelegten Ursprung 9 bestimmbar sind. Somit ergibt sich für jeden Punkt auf der Hornhaut 1 eine Koordinatenangabe, die den Ort des Punktes genau festlegt.

Darüber hinaus ist in Figur 1 eine erste Sollfläche 10 eingezeichnet, die vom behandelnden Arzt so variiert werden kann, daß Lage und Verlauf den klinischen Anforderungen entsprechen. Durch die Variation entsteht eine zweite Sollfläche 11, die der Arzt nach seiner klinischen Erfahrung relativ zur Augenhornhaut positioniert.

Aus der relativen Lage eines jeden Punktes 7 zur zweiten Sollfläche 11 errechnet der Rechner den Abstand 12 für jeden einzelnen Punkt. Dieser Abstand dient als Grundlage für die spätere Behandlung der Hornhaut 1.

In Figur 2 sind in einer Draufsicht durch unterschiedliche Schraffuren mögliche Krümmungen einer Augenhornhaut dargestellt. Enge Schraffuren 13, 14, 15 zeigen eine stärkere Krümmung, während hellere Schraffuren 16, 17 eine geringere Krümmung anzeigen. Anstelle von Schraffuren werden in der Praxis vorteilhafterweise Farben eingesetzt.

Alternativ oder ergänzend können statt der Krümmungen auch die Orte der Oberfläche der Augenhornhaut dargestellt werden.

Auch die Abstände 12 zwischen der Augenhornhaut 1 und der zweiten Sollfläche 11 können entsprechend Figur 2 durch Schraffuren oder Farben dargestellt werden, um Flächen, die besonders stark behandelt werden müssen, hervorzuheben.

Figur 3 zeigt schematisch die Behandlung einer Hornhautoberfläche gemäß Figur 1 mit einer Lasereinrichtung. Auf der Grundlage der Abstände zwischen der Hornhaut 1 und der zweiten Sollfläche 11 wurden vom Rechner Ort und Anzahl der Schüsse 18, 19, 20, 21 berechnet. Da die Lasereinrichtung jedoch nur diskontinuierliche Einstellungen ermöglicht, entsteht nach der Behandlung eine Istfläche 22 die der zweiten Sollfläche 11 nur angenähert ist. Um das zu erwartende Ergebnis vor Durchführung der Behandlung zu überprüfen und gegebenenfalls das Behandlungsverfahren noch zu korrigieren, wird die Behandlung zunächst am Rechner simuliert. Erst nach Durchspielen verschiedener Sollflächen und/oder Behandlungsverfahren wird schließlich vom behandelnden Arzt eine Sollfläche und ein Oberflächenbehandlungsverfahren festgelegt, das dem anvisierten Ergebnis am ehesten gerecht wird.

## Patentansprüche

1. Verfahren zur Bestimmung von Daten zur Behandlung der Hornhaut eines Auges,
- bei dem die Raumkoordinaten einer Vielzahl an Punkten (7) der Oberfläche (1) ermittelt werden,
- diese Raumkoordinaten als Datensatz in einen Rechner eingegeben werden,
- die Form und Lage der Sollfläche (10, 11) in den Rechner eingegeben wird,
- für die Vielzahl an Punkten (7) auf der Oberfläche (1) der Abstand (12) zur Sollfläche (10, 11) errechnet wird und
- die Raumkoordinaten und die Abstände (12) der Vielzahl an Punkten (7) als Datensatz zur Behandlung der Oberfläche (1) ausgegeben werden, ***dadurch gekennzeichnete, dass*** in weiteren Verfahrensschritten mit dem berechneten Datensatz zur Behandlung der Oberfläche ein Verfahren der eigentlichen Oberflächenbehandlung im Rechner simuliert wird, wobei eine zu erwartende Istfläche (22) berechnet wird, diese mit der vorgegebenen Sollfläche verglichen wird und bei unzureichender Annäherung der zu erwartenden Istfläche an die Sollfläche solange die Form und/oder Lage der Sollfläche variiert wird und anhand dieser neuen Sollfläche eine erneute Abstandsberechnung erfolgt, bis die zu erwartende Istfläche annähernd der Sollfläche entspricht.

2. Verfahren nach Anspruch 1, ***dadurch gekennzeichnet, dass*** die Oberfläche (1) eine spiegelnde Oberfläche ist, die mittels Topometrie vermessen wird.

3. Verfahren nach einem der vorhergehenden Ansprüche, ***dadurch gekennzeichnet, dass*** die Raumkoordinaten der Oberfläche (1) der Vielzahl an Punkten (7) iterativ als Koordinaten berechnet werden.

4. Verfahren nach einem der vorhergehenden Ansprüche, ***dadurch gekennzeichnet, dass*** die Abstände (12) der Vielzahl an Punkten (7) zur Sollfläche (11) graphisch dargestellt werden.

5. Verfahren nach einem der vorhergehenden Ansprüche, ***dadurch gekennzeichnet, dass*** unterschiedliche Abstände (12) durch unterschiedliche Farben dargestellt werden.

6. Verfahren nach einem der vorhergehenden Ansprüche, ***dadurch gekennzeichnet, dass*** die Form und/oder Lage der Sollfläche (10, 11) durch Raytracing bestimmt wird.

7. Verfahren nach einem der vorhergehenden Ansprüche, ***dadurch gekennzeichnet, dass*** das Ergebnis des mit dem Datensatz zur Behandlung der Oberfläche simulierten Oberflächenbehandlungsverfahrens angezeigt wird.

8. Verfahren nach einem der vorhergehenden Ansprüche, ***dadurch gekennzeichnet, dass*** das Oberflächenbehandlungsverfahren ein Laserverfahren ist.

## Claims

1. A method for determining data for treating the cornea,
- in which the spatial coordinates of a plurality of points (7) on the surface (1) are determined,
- these spatial coordinates are entered into a computer as a data record,
- the shape and location of the target surface, (10, 11), is input into the computer,
- the distance (12) to the target surface (10, 11) is calculated for the plurality of points (7) on the surface (1), and
- the spatial coordinates and the distances (12) of the plurality of points (7) are output as a data record for the treatment of the surface (1),
***characterized in that*** in further process steps with the calculated data record for .treatment of the surface, a method for actual surface treatment is simulated in the computer according to which an anticipated actual surface (22) is calculated and compared with the predefined target surface, and if the anticipated actual surface does not adequately approximate the target surface, the shape and/or position of the target surface is varied and a new distance calculation is made on the basis of this new target surface until the anticipated actual surface adequately approximates the target surface.

2. The method according to claim 1, ***characterized in that*** surface (1) is a reflective surface that is measured by topometry.

3. The method according to any of the preceding claims, ***characterized in that*** the spatial coordinates of surface (1) of plurality of points (7) are calculated iteratively as coordinates.

4. The method according to any of the preceding claims, ***characterized in that*** distances (12) of the plurality of points (7) to target surface (11) are represented graphically.

5. The method according to any of the preceding claims, ***characterized in that*** different distances (12) are represented by different colors.

6. The method according to any of the preceding claims, ***characterized in that*** the shape and/or position of target surface (10, 11) is determined by ray tracing.

7. The method according to any of the preceding claims, ***characterized in that*** the result of the surface treatment method simulated with the data record for treatment of the surface is displayed.

8. The method according to any of the preceding claims, ***characterized in that*** the surface treatment method is a laser method.

## Revendications

1. Procédé pour déterminer des données servant au traitement de la cornée d'un oeil,
- avec lequel les coordonnées dans l'espace d'un grand nombre de points (7) de la surface (1) sont déterminées,
- ces coordonnées dans l'espace sont entrées dans un ordinateur comme enregistrement,
- la forme et l'emplacement de la surface théorique (10, 11) sont entrés dans l'ordinateur,
- la distance (12) à la surface théorique (10, 11) est calculée pour la pluralité de points (7) sur la surface (1) et
- et les coordonnées dans l'espace et les distances (12) de la pluralité de points (7) sont éditées comme enregistrement pour le traitement de la surface (1), ***caractérisés en ce qu*'**un procédé du traitement de surface proprement dit est simulé dans l'ordinateur au cours d'autres étapes du procédé avec l'enregistrement calculé pour le traitement de la surface, moyennant quoi une surface réelle (22) à escompter est calculée, celle-ci étant comparée avec la surface théorique prédéfinie et dans le cas d'un rapprochement insuffisant de la surface réelle à escompter de la surface théorique aussi longtemps que la forme et/ou la position de la surface théorique sont modifiées et qu'un nouveau calcul de distance est effectué à l'aide de cette nouvelle surface théorique, jusqu'à ce que la surface réelle à escompter corresponde approximativement à la surface théorique.

2. Procédé selon la revendication 1, ***caractérisé en ce que*** la surface (1) est une surface réfléchissante, qui est mesurée par topométrie.

3. Procédé selon quelconque des revendications précédentes, ***caractérisé en ce que*** les coordonnées dans l'espace de la surface (1) de la pluralité de points (7) sont calculées par itération sous la forme de coordonnées.

4. Procédé selon l'une quelconque des revendications précédentes, ***caractérisé en ce que*** les distances (12) de la pluralité de points (7) à la surface théorique (11) sont représentées sous forme graphique.

5. Procédé selon l'une quelconque des revendications précédentes, ***caractérisé en ce que*** des distances (12) différentes sont représentées par des couleurs différentes.

6. Procédé selon l'une quelconque des revendications précédentes, ***caractérisé en ce que*** la forme et/ou la position de la surface théorique (10, 11) sont définies par le Raytracing.

7. Procédé selon l'une quelconque des revendications précédentes, ***caractérisé en ce que*** le résultat du procédé de traitement superficiel simulé avec l'enregistrement pour le traitement de la surface est affiché.

8. Procédé selon l'une quelconque des revendications précédentes, ***caractérisé en ce que*** le procédé du traitement de surface est un procédé au laser.
